(19) Europäisches Patentamt
European Patent Office
Office européen des brevets

(11) EP 1 087 838 B1

# (12) EUROPÄISCHE PATENTSCHRIFT

(45) Veröffentlichungstag und Bekanntmachung des Hinweises auf die Patenterteilung:
**25.05.2005 Patentblatt 2005/21**

(21) Anmeldenummer: **99910357.5**

(22) Anmeldetag: **18.03.1999**

(51) Int Cl.[7]: **B01J 31/22**, B01J 31/18, C07F 15/00, C08G 61/08

(86) Internationale Anmeldenummer:
**PCT/EP1999/001785**

(87) Internationale Veröffentlichungsnummer:
**WO 1999/051344 (14.10.1999 Gazette 1999/41)**

## (54) ALKYLIDENKOMPLEXE DES RUTHENIUMS MIT N-HETEROZYKLISCHEN CARBENLIGANDEN; VERWENDUNG ALS KATALYSATOREN FÜR DIE OLEFIN-METATHESE

ALKYLIDENE COMPLEXES OF RUTHENIUM WITH N-HETEROCYCLIC CARBENE LIGANDS AND THEIR USE AS CATALYSTS FOR OLEFIN METATHESIS

COMPLEXES ALKYLIDENE DE RUTHENIUM AVEC DES LIGANDS A BASE DE CARBENE N-HETEROCYCLIQUE, LEUR UTILISATION COMME CATALYSEUR DE METATHESE DES OLEFINES

(84) Benannte Vertragsstaaten:
**CH DE FR GB LI NL**

(30) Priorität: **06.04.1998 DE 19815275**

(43) Veröffentlichungstag der Anmeldung:
**04.04.2001 Patentblatt 2001/14**

(73) Patentinhaber: **Degussa AG
40474 Düsseldorf (DE)**

(72) Erfinder:
- **HERRMANN, Wolfgang, Anton
D-85354 Freising (DE)**
- **SCHATTENMANN, Wolfgang
D-81677 München (DE)**
- **WESKAMP, Thomas
D-80805 München (DE)**

(56) Entgegenhaltungen:
**EP-A- 0 721 953** **WO-A-97/06185**

- **T. WESKAMP ET AL.: "A Novel Class of Ruthenium Catalysts for Olefin metathesis" ANGEW. CHEM. INT. ED., Bd. 37, Nr. 18, 1998, Seiten 2490-2493, XP002103755**

EP 1 087 838 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft Alkylidenkomplexverbindungen des Rutheniums mit N-heterozyklischen Carbenliganden und ein Verfahren zur Herstellung von Olefinen durch Olefin-Metathese aus acyclischen Olefinen mit zwei oder mehr Kohlenstoffatomen oder/und aus cyclischen Olefinen mit vier oder mehr Kohlenstoffatomen, wobei mindestens eine dieser Alkylidenkomplexverbindungen als Katalysator eingesetzt wird.

Übergangsmetallkatalysierte C-C-Verknüpfungen gehören zu den wichtigsten Reaktionen der organischen Synthesechemie. In diesem Zusammenhang stellt die Olefin-Metathese einen wesentlichen Bestandteil dar, da mittels dieser Reaktion nebenproduktfrei Olefine synthetisiert werden können. Die Olefin-Metathese besitzt dabei nicht nur hohes Potential auf dem Sektor der präparativen, organischen Synthese (RCM, Ethenolyse, Metathese acyclischer Olefine), sondern auch in der Polymerchemie (ROMP, ADMET, Alkinpolymerisation). Seit ihrer Entdeckung in den 50er Jahren konnten mehrere großtechnische Prozesse realisiert werden. Dennoch avancierte die Olefin-Metathese erst in jüngster Zeit durch die Entdeckung neuer Katalysatoren zu einer breit anwendbaren Synthesemethode (J. C. Mol in: B. Cornils, W. A. Herrmann: Applied Homogeneous Catalysis with Organometallic Compounds, VCH, Weinheim, 1996, S.318-332; M. Schuster, S. Blechert, Angew. Chem. 1997, 109, 2124-2144; Angew. Chem. Int. Ed. Engl. 1997, 36, 2036-2056).

Zahlreiche, grundlegende Arbeiten haben wesentlich zum Verständnis dieser übergangsmetallkatalysierten Reaktion beigetragen, bei der ein Austausch von Alkylideneinheiten zwischen Olefinen erfolgt. Der allgemein akzeptierte Mechanismus beinhaltet Metallalkylidenkomplexe als aktive Spezien. Diese reagieren mit Olefinen zu Metallacyclobutanintermediaten, die unter Cycloreversion wieder Olefine und Alkylidenkomplexe generieren. Die Isolierung von metatheseaktiven Alkyliden- und Metallacyclobutankomplexen untermauert diese mechanistischen Vorstellungen.

Zahlreiche Beispiele finden sich vor allem in der Komplexchemie des Molybdäns und Wolframs. Speziell durch Arbeiten von Schrock wurden wohldefinierte Alkylidenkomplexe erhalten, die in ihrer Reaktivität kontrollierbar sind (J. S. Murdzek, R. R. Schrock, Organometallics 1987, 6, 1373-1374). Die Einführung einer chiralen Ligandsphäre an diesen Komplexen ermöglichte die Synthese von Polymeren mit hoher Taktizität (K. M. Totland, T. J. Boyd, G. C. Lavoie, W. M. Davis, R. R. Schrock, Macromolecules 1996, 29, 6114-6125). Chirale Komplexe gleichen Strukturtyps wurden auch in der Ringschluß-Metathese mit Erfolg eingesetzt (O. Fujimura, F. J. d. l. Mata, R. H. Grubbs, Organometallics 1996, 15, 1865-1871). Nachteilig stellt sich jedoch die hohe Empfindlichkeit gegenüber funktionellen Gruppen, Luft und Wasser heraus.

In jüngster Zeit haben sich phosphanhaltige Komplexsysteme des Rutheniums etabliert (R. H. Grubbs, S. T. Nguyen, L. K. Johnson, M. A. Hillmyer, G. C. Fu, WO 96/04289, 1994; P. Schwab, M. B. France, J. W. Ziller, R. H. Grubbs, Angew. Chem., 1995,107, 2179-2181; Angew. Chem. Int. Ed. Engl. 1995, 34, 2039-2041). Aufgrund des elektronenreichen, "weichen" Charakters später Übergangsmetalle besitzen diese Komplexe eine hohe Toleranz gegenüber harten, funktionellen Gruppen. Dies wird beispielsweise durch ihren Einsatz in der Naturstoffchemie (RCM von Dienen) demonstriert (Z. Yang, Y. He, D. Vourloumis, H. Vallberg, K. C. Nicolaou, Angew. Chem. 1997, 109, 170-172; Angew. Chem., Int. Ed. Engl. 1997, 36, 166-168; D. Meng, P. Bertinato, A. Balog, D. S. Su, T. Kamenecka, E. J. Sorensen, S. J. Danishefsky, J. Am. Chem. Soc. 1997, 119, 2733-2734; D. Schinzer, A. Limberg, A. Bauer, O. M. Böhm, M. Cordes, Angew. Chem. 1997, 109, 543-544; Angew. Chem., Int. Ed. Engl. 1997, 36, 523-524; A. Fürstner, K. Langemann, J. Am. Chem. Soc. 1997, 119, 9130-9136).

Die Variationsbreite der verwendeten Phosphanliganden ist jedoch aufgrund sterischer und elektronischer Faktoren sehr begrenzt. Lediglich stark basische, sterisch anspruchsvolle Alkylphosphane wie Tricyclohexyl-, Triisopropyl- und Tricyclopentylphosphan eignen sich für die Metathese acyclischer Olefine und wenig gespannter Ringsysteme. Demnach sind diese Katalysatoren nicht in ihrer Reaktivität einstellbar. Auch chirale Komplexe dieses Strukturtyps konnten nicht realisiert werden.

Aus diesen Gründen bestand die Aufgabe, maßgeschneiderte Metathesekatalysatoren zu entwickeln, die sich neben hoher Toleranz gegenüber funktionellen Gruppen durch eine variable Ligandensphäre auszeichnen und die eine Feineinstellung des Katalysators für spezielle Eigenschaften unterschiedlicher Olefine ermöglichen.

Die Aufgabe wird erfindungsgemäß gelöst durch eine Komplexverbindung des Rutheniums der allgemeinen Strukturformel I,

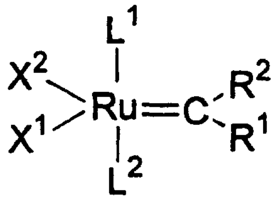

I

in der $X^1$ und $X^2$ gleich oder verschieden voneinander einen anionischen Liganden bedeuten,
in der $R^1$ und $R^2$ gleich oder unabhängig voneinander verschieden sind, aber auch einen Cyclus aufweisen können,
in der $R^1$ und $R^2$ für Wasserstoff oder/und für eine Kohlenwasserstoffgruppe stehen,
wobei die Kohlenwasserstoffgruppen gleich oder unabhängig voneinander verschieden aus geradkettigen, verzweigten, cyclischen oder/und nicht cyclischen Resten aus der Gruppe von Alkylresten mit 1 bis 50 Kohlenstoffatomen, Alkenylresten mit 1 bis 50 Kohlenstoffatomen, Alkinylresten mit 1 bis 50 Kohlenstoffatomen, Arylresten mit 1 bis 30 Kohlenstoffatomen und Silylresten bestehen,
wobei in den Kohlenwasserstoff- oder/und Silylgruppen die Wasserstoffatome teilweise oder gänzlich durch eine Alkyl-, Aryl-, Alkenyl-, Alkinyl-, Metallocenyl-, Halogen-, Nitro-, Nitrose-, Hydroxy-, Alkoxy-, Aryloxy-, Amino-, Amido-, Carboxyl-, Carbonyl-, Thio- oder/und Sulfongruppe einfach oder mehrfach, gleich oder unabhängig voneinander verschieden ersetzt sein können, in der der Ligand $L^1$ ein N-heterozyklisches Carben der allgemeinen Formel II - V ist und in der der Ligand $L^2$ ein Phosphan ist,

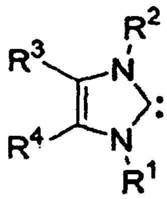

II

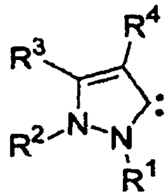

III

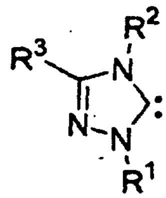

IV

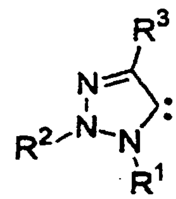

V

wobei $R^1$, $R^2$, $R^3$ und $R^4$ in den Formeln II, III, IV und V gleich oder verschieden für Wasserstoff oder/und für Kohlenwasserstoffgruppen stehen,
wobei die Kohlenwasserstoffgruppen aus gleichen oder verschiedenen, cyclischen, nicht cyclischen , geradkettigen oder/und verzweigten Resten aus der Gruppe von Alkylresten mit 1 bis 50 Kohlenstoffatomen, Alkenylresten mit 1 bis 50 Kohlenstoffatomen, Alkinylresten mit 1 bis 50 Kohlenstoffatomen und Arylresten mit 1 bis 30 Kohlenstoffatomen bestehen, bei denen gegebenenfalls mindestens ein Wasserstoff durch funktionelle Gruppen ersetzt sein kann, und wobei gegebenenfalls $R^3$ und $R^4$ für Halogen-, Nitro-, Nitrose-, Alkoxy-, Aryloxy-, Amino-, Carboxyl-, Carbonyl-, Thio- oder/und Sulfonylgruppen einfach oder mehrfach, gleich oder unabhängig voneinander verschieden stehen kann.

Vorzugsweise weisen die Alkylreste, Alkenylreste bzw. Alkinylreste in den Formeln I bis V 1 bis 20 Kohlenstoffatome, besonders bevorzugt 1 bis 12 Kohlenstoffatome, auf.

Die erfindungsgemäßen Komplexverbindungen sind hochaktive Katalysatoren für die Olefin-Metathese. Sie sind besonders kostengünstig. Die Olefin-Metathese mit den erfindungsgemäßen Katalysatoren zeichnet sich neben einer hohen Toleranz gegenüber unterschiedlichsten funktionellen Gruppen auch durch ihre Variationsvielfalt in der Ligandensphäre aus. Durch Variation der präparativ einfach zugänglichen N-heterozyklischen Carbenliganden können Aktivität und Selektivität gezielt gesteuert, und darüber hinaus kann Chiralität auf einfache Art und Weise eingeführt werden.

Vorzugsweise sind die anionischen Liganden $X^1$ und $X^2$ der erfindungsgemäßen Komplexverbindung gleich oder verschieden Halogenid, Pseudohalogenid, Tetraphenylborat, perhalogeniertes Tetraphenylborat, Tetrahalogenoborat, Hexahalogenophosphat, Hexahalogenoantimonat, Trihalogenomethansulfonat, Alkoxid, Carboxylat, Tetrahalogenoaluminat, Tertracarbonyl-Cobaltat, Hexahalogenoferrat(III), Tetrahalogenoferrat(III) oder/und Tetrahalogenopalladat(II), wobei Halogenid, Pseudohalogenid, Tetraphenylborat, perfluoriertes Tetraphenylborat, Tetrafluoroborat, Hexafluorophosphat, Hexafluoroantimonat, Trifluormethansulfonat, Alkoxid, Carboxylat, Tetrachloroaluminat, Tertracarbonyl-Cobaltat, Hexafluoroferrat(III), Tetrachloroferrat(III) oder/und Tetrachloropalladat(II) bevorzugt sind und wobei unter den Pseudohalogeniden Cyanid, Rhodanid, Cyanat, Isocyanat, Thiocyanat und Isothiocyanat bevorzugt sind.

In den allgemeinen Formeln **II, III, IV** und **V** kann der Wasserstoff in den Kohlenwasserstoffgruppen $R^1$, $R^2$, $R^3$ und $R^4$ teilweise oder gänzlich durch Halogen-, Nitro-, Nitroso-, Hydroxy-, Alkoxy-, Aryloxy-, Amino-, Amido-, Carboxyl-, Carbonyl-, Thio-, Sulfonyl- oder/und Metallocenylgruppen einfach oder mehrfach, gleich oder unabhängig voneinander verschieden ersetzt sein. In diesen Formeln kann $R^3$ und $R^4$ ein anneliertes Ringsystem darstellen.

Die Liganden $L^1$ und $L^2$ der Komplexverbindung der allgemeinen Strukturformel I können einen Chelatliganden der allgemeinen Formel **VI**

$$L^1—Y—L^2$$

**VI**

ausbilden, wobei die mit **Y** bezeichneten Brückenglieder aus cyclischen, nicht cyclischen, geradkettigen oder/und verzweigten Resten aus der Gruppe von Alkylenresten mit 1 bis 50 Kohlenstoffatomen, Alkenylenresten mit 1 bis 50 Kohlenstoffatomen, Alkinylenresten mit 1 bis 50 Kohlenstoffatomen, Arylenresten mit 1 bis 30 Kohlenstoffatomen, Metallocenylen-, Borylen- und Silylenresten bestehen können, bei denen gegebenenfalls mindestens ein Wasserstoff durch Alkyl-, Aryl-, Alkenyl-, Alkinyl-, Metallocenyl-, Halogen-, Nitro-, Nitroso-, Hydroxo-, Alkoxy-, Aryloxy-, Amino-, Amido-, Carboxyl-, Carbonyl-, Thio- oder/und Sulfonylgruppen, bevorzugt durch Alkyl-, Aryl- oder/und Metallocenylgruppen einfach oder mehrfach, gleich oder unabhängig voneinander verschieden substituiert sein kann.

Die Liganden der allgemeinen-Formeln **II, III, IV, V** oder/und **VI** können zentrale, axiale oder/und planare Chiralität aufweisen.

In der allgemeinen Strukturformel **I** der Komplexverbindung stehen $R^1$ bis $R^2$ bevorzugt für Wasserstoff, substituierte oder/und nichtsubstituierte Alkyl-, Alkenyloder/und Arylreste, $X^1$ und $X^2$ bevorzugt für Halogenid-, Alkoxid- oder/und Carboxylationen sind und $L^1$ bevorzugt für ein N-heterozyklisches Carben der allgemeinen Formel **II.**

Die Synthese der Komplexe erfolgt üblicherweise durch Ligandsubstitution entsprechender Phosphankomplexe. Diese können entsprechend Reaktionsgleichung (1) selektiv zweifach oder entsprechend Reaktionsgleichung (2) einfach substituiert werden. Im Fall der einfachen Substitution kann das zweite Phosphan selektiv durch einen anderen Elektronendonor, z. B. Pyridin, Phosphan, N-Heterozyklencarben, Phosphit, Stibin, Arsin substituiert werden entsprechend Reaktionsgleichung (3).

Insbesondere gelingt auf diesem Weg die erstmalige Darstellung von chiralen, metatheseaktiven Katalysatoren auf Rutheniumbasis (Komplexbeispiele 2 und 3).

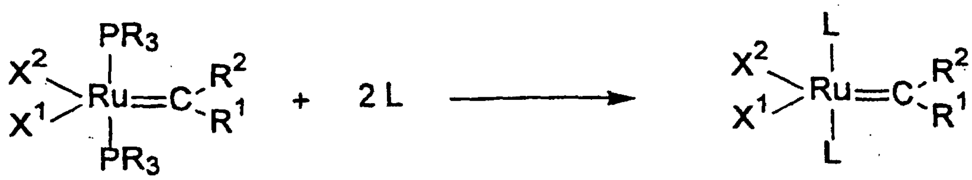

(1)

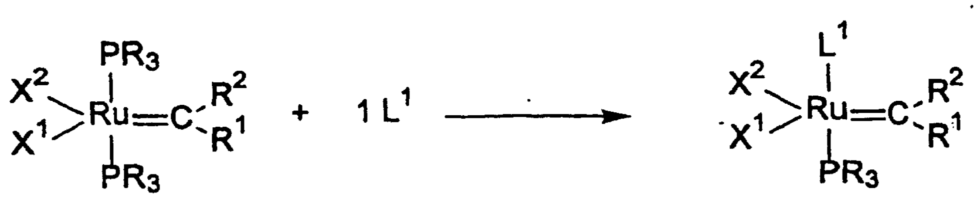

(2)

Die erfindungsgemäßen Komplexverbindungen erweisen sich als äußerst effiziente Katalysatoren in der Olefin-Metathese. Die ausgezeichnete Metatheseaktivität wird durch mehrere Beispiele verschiedener metathetischer Reaktionen in den Beispielen demonstriert.

Deshalb umfaßt diese Erfindung auch die Verfahren aller Olefin-Metathese-Reaktionen wie Ringöffnende Metathesepolymerisation (ROMP), Metathese acyclischer Olefine, Ethenolyse, Ringschlußmetathese (RCM), acyclische Dien-Metathese-Polymerisation (ADMET) und Depolymerisation olefinischer Polymere. Die hohe Stabilität und Toleranz der erfindungsgemäßen Komplexverbindungen gegenüber funktionellen Gruppen, insbesondere Gruppen von Alkoholen,

Aminen, Thiolen, Ketonen, Aldehyden, Carbonsäuren, Estern, Amiden, Ethem, Silanen, Sulfiden und Halogenen erlaubt die Anwesenheit derartiger funktioneller Gruppen während der Metathesereaktion.

Die Aufgabe wird ferner durch ein Verfahren zur Herstellung von acyclischen Olefinen mit zwei oder mehr Kohlenstoffatomen oder/und von cyclischen Olefinen mit vier oder mehr Kohlenstoffatomen jeweils entsprechend der allgemeinen Formel **VII**

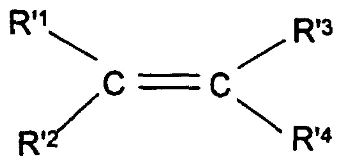

VII

aus acyclischen Olefinen mit zwei oder mehr Kohlenstoffatomen oder/und aus cyclischen Olefinen mit vier oder mehr Kohlenstoffatomen jeweils entsprechend der allgemeinen Formel **VII** durch Olefin-Metathese-Reaktion in Gegenwart mindestens eines Katalysators gelöst, das dadurch gekennzeichnet ist, daß

ein Katalysator nach einem der Ansprüche 1 bis 7 eingesetzt wird und

daß $R'^1$, $R'^2$, $R'^3$ und $R'^4$ in der allgemeinen Formel **VII** für Wasserstoff oder/und Kohlenwasserstoffgruppen stehen, wobei die Kohlenwasserstoffgruppe aus gleich oder unabhängig voneinander verschieden geradkettigen, verzweigten, cyclischen oder/und nicht cyclischen Resten aus der Gruppe von Alkylresten mit 1 bis 50 Kohlenstoffatomen, Alkenylresten mit 1 bis 50 Kohlenstoffatomen, Alkinylresten mit 1 bis 50 Kohlenstoffatomen, Arylresten mit 1 bis 30 Kohlenstoffatomen, Metallocenyloder/und Silylresten besteht, bei denen gegebenenfalls mindestens ein Wasserstoff durch eine funktionelle Gruppe ersetzt sein kann,

wobei gegebenenfalls $R'^1$, $R'^2$, $R'^3$ und $R'^4$ für Halogen-, Nitro-, Nitroso-, Hxdroxy-, Alkoxy-, Aryloxy-, Amino-, Amido-, Carboxyl-, Carbonyl-, Thio-, Sulfonyl- oder/und Metallocenylgruppen einfach oder mehrfach, gleich oder unabhängig voneinander verschieden stehen.

Vorzugsweise sind in den eingesetzten Olefinen eine oder mehrere Doppelbindungen enthalten. Insbesondere bilden $R'^1$, $R'^2$, $R'^3$ und $R'^4$ in den herzustellenden Olefinen der allgemeinen Formel **VII** paarweise, einfach oder mehrfach, gleich oder unabhängig voneinander verschieden einen Cyclus aus.

Vorzugsweise ist in den herzustellenden Olefinen der allgemeinen Formel **VII** der Wasserstoff in den Kohlenwasserstoffgruppen $R'^1$, $R'^2$, $R'^3$ und $R'^4$ teilweise oder gänzlich durch Halogen-, Silyl-, Nitro-, Nitrose-, Hydroxy-, Alkoxy-, Aryloxy-, Amino-, Amido-, Carboxyl-, Carbonyl-, Thio-, Sulfonyl- oder/und Metallocenylgruppen einfach oder mehrfach, gleich oder unabhängig voneinander verschieden ersetzt.

Bei dem erfindungsgemäßen Verfahren kann der Prozeß mit oder ohne Lösungsmittel, jedoch vorzugsweise mit organischen Lösungsmitteln, durchgeführt werden. Vorzugsweise kann das erfindungsgemäße Verfahren unter Zusatz einer Brönstedt-Säure, vorzugsweise von HCl, HBr, HI, $HBF_4$, $HPF_6$ oder/und Trifluoressigsäure, oder/und unter Zusatz einer Lewis-Säure, vorzugsweise von $BF_3$, $AlCl_3$ oder/und $ZnI_2$, durchgeführt werden.

Damit wird es erstmals überraschenderweise möglich, die verschiedensten Olefine individuell auf unterschiedliche Eigenschaften aufgrund geringer Variation der Katalysebedingungen oder/und der Katalysatoren maßzuschneidern, da das erfindungsgemäße Verfahren zur Herstellung von Olefinen eine unerwartet hohe Toleranz gegenüber funktionellen Gruppen aufweist.

Beispiele:

Die folgenden Beispiele belegen die Erfindung, schränken sie aber nicht in ihrer Breite ein.

1) Herstellung der erfindungsgemäßen Komplexverbindung

Allgemeine Arbeitsvorschrift:

1 mmol $(PPh_3)_2Cl_2Ru(=CHPh)$ wurden in 20 ml Toluol gelöst und mit einer Lösung von 2.2 equiv des entsprechenden Imidazolin-2-ylidens in 5 ml Toluol versetzt. Die Reaktionslösung wurde 45 min bei Raumtemperatur RT gerührt, anschließend auf ca. 2 ml eingeengt, und das Rohprodukt wurde mit 25 ml Pentan ausgefällt. Das Rohprodukt wurde mehrmals in 2 ml Toluol aufgenommen und mit 25 ml Pentan ausgefällt. Der Rückstand wurde mit Toluol extrahiert, die Lösung zur Trockene eingeengt, mit Pentan zweimal gewaschen und mehrere Stunden im Hochvakuum

getrocknet.

Zur Charakterisierung werden größtenteils die Daten von Tieftemperatur-NMR-Spektren angegeben, da die Spektren bei Raumtemperatur aufgrund dynamischer Effekte teilweise nicht die gesamte Information enthalten.

Nach der angegebenen allgemeinen Arbeitsvorschrift werden folgende Verbindungen dargestellt:

1a) Benzyliden-dichloro-bis(1,3-diisopropylimidazolin-2-yliden)-ruthenium - Komplexverbindung 1:

Ausbeute: 487 mg (0.86 mmol = 86 % der Theorie)

| | | | | |
|---|---|---|---|---|
| Elementaranalyse **EA** für $C_{25}H_{38}Cl_2N_4Ru$ (566.58) | gefunden | C 53.21 | H 6.83 | N 9.94; |
| | berechnet | C 53.00 | H 6.76 | N 9.89. |

**$^1$H-NMR** ($CD_2Cl_2$/ 200 K): δ 20.33 (1H, s, Ru=CH), 8.25 (2H, d, $^3J_{HH}$ = 7.6 Hz, *o*-H von $C_6H_5$), 7.63 (1 H, t, $^3J_{HH}$= 7.6 Hz, *p*-H von $C_6H_5$), 7.34 (2H, t, *m*-H von $C_6H_5$, $^3J_{HH}$ = 7.6 Hz), 7.15 (2H, br, NCH), 7.03 (2H, br, NCH), 5.97 (2H, spt, $^3J_{HH}$= 6.4 Hz, NC*H*Me$_2$), 3.73 (2H, spt, $^3J_{HH}$= 6.4 Hz, NC*H*Me$_2$), 1.64 (12H, d, $^3J_{HH}$= 6.4 Hz, NCH*Me*$_2$), 1.11 (6H, d, $^3J_{HH}$= 6.4 Hz, NCH*Me*$_2$), 0.75 (6H, d, $^3J_{HH}$= 6.4 Hz, NCH*Me*$_2$).

**$^{13}$C-NMR** ($CD_2Cl_2$/ 200 K): δ 295.6 (Ru=CH), 183.5 (NCN), 151.6 (*ipso*-C von $C_6H_5$), 129.5,128.6 und 128.1 (*o*-C, *m*-C und *p*-C von $C_6H_5$), 118.1 und 117.2 (NCH), 52.1 und 50.1 (N*C*HMe$_2$), 24.5, 23.8, 23.8 und 22.4 (NCH*Me*$_2$).

1b) Benzyliden-dichloro-bis(1,3-di-((R)-1'-phenylethyl)imidazolin-2-yliden)-ruthenium - Komplexverbindung 2:

Ausbeute: 676 mg (0.83 mmol = 83 % d. Th.)

| | | | | |
|---|---|---|---|---|
| **EA** für $C_{45}H_{46}Cl_2N_4Ru$ (814.86) | gef. | C 66.48 | H 5.90 | N 6.73; |
| | ber. | C 66.33 | H 5.69 | N 6.88. |

**$^1$H-NMR** ($CD_2Cl_2$/ 200 K): δ 20.26 (1 H, s, Ru=CH), 8.13 (2H, br, *o*-H $C_6H_5$), 7.78 - 6.67 (29H, davon 2*m*-H und 1*p*-H von $C_6H_5$, 20H von NCHMe*Ph*, 2H von NC*H*MePh und 4H von NCH), 4.91 (2H, m, NC*H*MePh), 1.84 (3H, d, $^3J_{HH}$ = 6.6 Hz, NCH*Me*Ph), 1.81 (3H, d, $^3J_{HH}$ = 6.6 Hz, NCH*Me*Ph), 1.51 (3H, d, $^3J_{HH}$ = 6.6 Hz, NCH*Me*Ph), 1.21 (3H, d, $^3J_{HH}$ = 6.6 Hz, NCH*Me*Ph).

**$^{13}$C-NMR** ($CD_2Cl_2$/ 200 K): δ 294.7 (Ru=CH), 186.0 und 185.6 (NCN), 151.2 (*ipso*-C von $C_6H_5$), 141.2, 140.3, 140.1 und 139.9 (ipso-C von NCHMe*Ph*), 133.1 -125.9 (*o*-C, *m*-C, *p*-C von $C_6H_5$ und NCHMe*Ph*), 120.5, 119.9, 119.2 und 118.8 (NCH), 57.6, 57.4, 56.7 und 56.1 (N*C*HMePh), 22.2, 20.6, 20.4 und 20.3 (NCH*Me*Ph).

1c) Benzyliden-dichloro-bis(1,3-di-((R)-1'-naphtylethyl)imidazalin-2-yliden)-ruthenium-Komplexverbindung 3:

Ausbeute: 792 mg (0.78 mmol = 78 % d. Th.)

| | | | | |
|---|---|---|---|---|
| **EA** für $C_{61}H_{54}Cl_2N_4Ru$ (1015.1) | gef. | C 72.34 | H 5.46 | N 5.45; |
| | ber. | C 72.18 | H 5.36 | N 5.52. |

**$^1$H-NMR** ($CD_2Cl_2$/260 K): δ 20.90 (1H, s, Ru=CH), 8.99 (2H, br, *o*-H von $C_6H_5$), 8.2 - 5.6 (39H, davon 2*m*-H und 1*p*-H von $C_6H_5$, 28H von NCHMe*Naph*, 4H von NCH und 4H von NC*H*MeNaph), 2.5 - 0.8 (12H, m, NCH*Me*Naph).

**$^{13}$C-NMR** ($CD_2Cl_2$/ 260 K): δ 299.9 (Ru=CH), 187.2 und 184.7 (NCN), 152.0 (*ipso*-C von $C_6H_5$), 136.0 - 124.0 (*o*-C, *m*-C, *p*-C von $C_6H_5$ und NCHMe*Naph*), 121.7, 121.0, 119.9 und 118.9 (NCH), 56.7, 56.1, 55.0 und 54.7 (N*C*HMeNaph), 24.7, 24.3, 21.0 und 20.0 (NCH*Me*Naph).

Für die folgenden Komplexe sind geringfügige Abweichungen von der allgemeinen Arbeitsvorschrift notwendig:

1d) (4-Chlorbenzyliden)-dichloro-bis(1,3-diisopropylimidazolin-2-yliden)-ruthenium - Komplexverbindung **4:**

Als Edukt wurde 1 mmol $(PPh_3)_2Cl_2Ru[=CH(p-C_6H_4Cl)]$ eingesetzt. Die weitere Vorgehensweise entsprach der allgemeinen Arbeitsvorschrift.
Ausbeute: 535 mg (0.89 mmol = 89 % d. Th.)

| | | | | |
|---|---|---|---|---|
| **EA** für $C_{24}H_{38}Cl_3N_4Ru$ (601.03) | gef. | C 48.13 | H 6.33 | N 9.24; |
| | ber. | C 47.96 | H 6.37 | N 9.32. |

**$^1$H-NMR** ($CD_2Cl_2$/ 200 K): δ 20.33 (1H, s, Ru=CH), 8.25 (2H, d, $^3J_{HH}$ = 7.6 Hz, *o*-H von $C_6H_4Cl$), 7.63 (1H, t, $^3J_{HH}$ = 7.6 Hz, *m*-H von $C_6H_4Cl$), 7.15 (2H, br, NCH), 7.03 (2H, br, NCH), 5.97 (2H, spt, $^3J_{HH}$ = 6.4 Hz, NC*H*Me$_2$), 3.73 (2H, spt, $^3J_{HH}$ = 6.4 Hz, NC*H*Me$_2$), 1.64 (12H, d, $^3J_{HH}$ = 6.4 Hz, NCH*Me*$_2$), 1.11 (6H, d, $^3J_{HH}$ = 6.4 Hz, NC*H*Me$_2$), 0.75 (6H, d, $^3J_{HH}$ = 6.4 Hz, NCH*Me*$_2$).
**$^{13}$C-NMR** ($CD_2Cl_2$/ 200 K): δ 295.6 (Ru=CH), 183.5 (NCN), 151.6 (ipso-C von $C_6H_4Cl$), 134.3 (*p*-C von $C_6H_4Cl$), 128.6 und 128.1 (*o*-C und *m*-C von $C_6H_4Cl$), 118.1 und 117.2 (NCH), 52.1 und 50.1 (N*C*HMe$_2$), 24.5, 23.8, 23.8 und 22.4 (NCH*Me*$_2$).

1e) Benzyliden-dichloro-bis(1,3-dicyclohexylimidazolin-2-yliden)-ruthenium - Komplexverbindung 5:

1 mmol $(PPh_3)_2Cl_2Ru(=CHPh)$ wurden in 25 ml Toluol gelöst und mit einer Lösung von 2.2 equiv. 1,3-Dicyclohexylimidazolin-2-yliden in 5 ml Toluol versetzt. Die Reaktionslösung wurde 45 min bei RT gerührt und anschließend vom Lösungsmittel befreit. Anders als bei der allgemeinen Arbeitsvorschrift wurde das Rohprodukt durch Flashchromatographie gereinigt.
Ausbeute: 305 mg (0.42 mmol = 42 % d. Th.)

| | | | | |
|---|---|---|---|---|
| **EA** für $C_{37}H_{54}Cl_2N_4Ru$ (726.84) | gef. | C 61.23 | H 7.56 | N 7.87; |
| | ber. | C 61.14 | H 7.49 | N 7.71. |

**$^1$H-NMR** ($CD_2Cl_2$/ 298 K): δ 20.45 (1H, s, Ru=CH), 8.31 (2H, d, $^3J_{HH}$ = 7.6 Hz, *o*-Hvon $C_6H_5$), 7.63 (1H, t, $^3J_{HH}$ = 7.6 Hz, *p*-H- von $C_6H_5$), 7.34 (2H, t, $^3J_{HH}$ = 7.6 Hz, *m*-H- von $C_6H_5$), 7.14 (2H, br, NCH), 7.00 (2H, br, NCH), 6.06 (2H, br, CH von $NC_6H_{11}$), 3.82 (2H, br, CH von $NC_6H_{11}$), 1.64 (12H, br, $CH_2$ von $NC_6H_{11}$), 0.93 (12H, br, $CH_2$ von $NC_6H_{11}$).
**$^{13}$C-NMR** ($CD_2Cl_2$/ 298 K): δ 299.4 (Ru=CH), 182.9 (NCN), 152.0 (*ipso*-C von $C_6H_5$), 131.1, 129.8 und 129.1 (*o*-C, *m*-C und *p*-C von $C_6H_5$), 118.3 und 117.8 (br, NCH), 59.6 und 57.5 (br, CH von $NC_6H_{11}$), 35.7, 26.9 und 25.6 (br, $CH_2$ von $NC_6H_{11}$).

1f) Benzyliden-dichloro-(1,3-di-*tert.*-butylimidazolin-2-yliden)-(triphenyl-phosphin)-ruthenium - Komplexverbindung 6:

1 mmol $(PPh_3)_2Cl_2Ru(=CHPh)$ wurden in 20 ml Toluol gelöst und mit einer Lösung von 1.1 equiv. 1,3-Di-*tert.*-butylimidazolin-2-yliden in 5 ml Toluol versetzt. Die Reaktionslösung wurde 30 min bei RT gerührt, anschließend auf ca. 2 ml eingeengt, und das Rohprodukt wurde mit 25 ml Pentan ausgefällt. Die weitere Aufarbeitung erfolgte gemäß der allgemeinen Arbeitsvorschrift.
Ausbeute: 493 mg (0.70 mmol = 70% d. Th.)

| | | | | | |
|---|---|---|---|---|---|
| **EA** für $C_{36}H_{41}Cl_2N_2P_1Ru$ (704.69) | gef. | C 61.12 | H 5.55 | N 3.62 | P 4.59 |
| | ber. | C 61.36 | H 5.86 | N 3.98 | P 4.38. |

**$^1$H-NMR** ($CD_2Cl_2$/ 200 K): δ 20.70 (1 H, s, Ru=CH), 8.03 (2H, d, $^3J_{HH}$ = 7.6 Hz, *o*-H von $C_6H_5$), 7.50 - 6.95 (20H, davon 2*m*-H und 1*p*-H von $C_6H_5$, 15H von $PPh_3$ und 2H von NCH), 1.86 (9H, s, $NCMe_3$), 1.45 (9H, s, $NCMe_3$).
**$^{13}$C-NMR** ($CD_2Cl_2$/ 200 K): δ 307.4 (br, Ru=CH), 178.3 (d, $J_{PC}$ = 86 Hz, NCN), 151.5 (d, $J_{PC}$ = 4.5 Hz, *ipso*-C von $C_6H_5$), 135.0 (m, *o*-C von $PPh_3$), 131.9 (m, *ipso*-C von $PPh_3$), 130.2 (s, *p*-C von $PPh_3$), 129.5, 128.6 und 128.1 (s, *o*-C, *m*-C und *p*-C von $C_6H_5$), 128.0 (m, *m*-C von $PPh_3$), 117.7 und 117.6 (NCH), 58.7 und 58.5 ($NCMe_3$), 30.0 und 29.5 (NC*Me*$_3$).
**$^{31}$P-NMR** ($CD_2Cl_2$/ 200 K): δ 40.7 (s, $PPh_3$).

1g) Benzyliden-dichloro-(1,3-di-cyclohexyl-imidazolin-2-yliden)-(tricyclohexylphosphin)-ruthenium

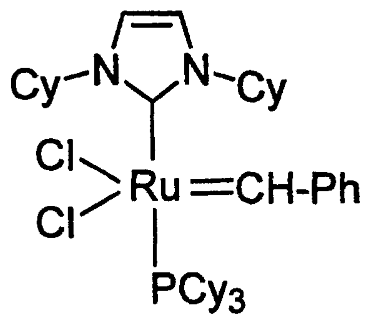

1 mmol $RuCl_2(PCy_3)_2(CHPh)$ in 100 mL THF werden bei -78 °C tropfenweise mit einer Lösung von 1.2 mmol Dicyclohexylimidazolin-2-yliden versetzt. Es wird im Laufe von 5 h langsam auf Raumtemperatur erwärmt und anschließend das Lösungsmittel entfernt. Man extrahiert das Rohprodukt mit einem Gemisch aus 2mL Toluol und 25 mL Pentan und fällt das Produkt bei - 78 °C aus dieser Lösung aus.
Ausbeute: 0.80 mmol (80 % d. Th.)
**EA** für $C_{40}H_{63}Cl_2N_2PRu$: ber. C 61.99, H 8.20, N 3.62; gef. C 61.11; H 8.29; N 3.59.
**$^1$H NMR** ($CD_2Cl_2$ / 25 °C): δ = 20.30 (1 H, d, $^3J_{PH}$ = 7.4 Hz, Ru=CH), 8.33 (2H, d, $^3J_{HH}$ = 7.4 Hz, *o*-H of $C_6H_5$), 7.62 (1H, t, $^3J_{HH}$ = 7.4 Hz, *p*-H of $C_6H_5$), 7.33 (2H, t, $^3J_{HH}$ = 7.4 Hz, *m*-H of $C_6H_5$), 7.11 (1H, s, NCH), 6.92 (1H, s, NCH), 5.97 (1H, m, CH of $NC_6H_{11}$), 3.36 (1H, m, CH of $NC_6H_{11}$), 2.42 (3H, m, CH of $PCy_3$), 1.90-0.89 (50H, all m, $CH_2$ of $NC_6H_{11}$ and $PCy_3$).
**$^{13}$C NMR** ($CD_2Cl_2$ / 25 °C): δ = 298.7 (Ru=CH), 181.2 (d, $J_{PC}$ = 88 Hz, NCN), 152.5 (*ipso*-C of $C_6H_5$), 130.8, 129.8, and 129.2 (*o*-C, *m*-C, and *p*-C of $C_6H_5$), 118.9 and 118.0 (NCH), 59.5 and 57.7 (CH of $NC_6H_{11}$) 33.2 (d, $J_{PC}$ = 17 Hz, *ipso*-C of $PCy_3$), 29.9 (s, *m*-C of $PCy_3$), 26.8 (d, $J_{PC}$ = 3.7 Hz, *o*-C of $PCy_3$), 25.4 (s, *p*-C of $PCy_3$) 34.9, 33.3, 33.1, 28.2, 28.1, and 25.7 ($CH_2$ of $NC_6H_{11}$).
**$^{31}$P NMR** ($CD_2Cl_2$ / 25 °C): δ = 28.2.

1h) Benzyliden-dichloro-(1,3-di-((*R*)-1'-phenylethyl)imidazolin-2-yliden)(tricyclohexylphosphin)-ruthenium

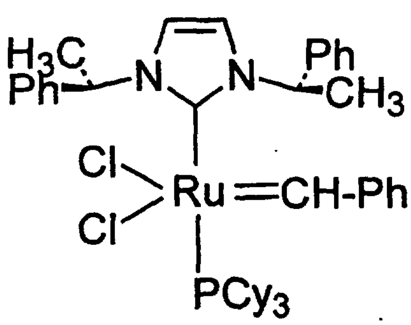

1 mmol $RuCl_2(PCy_3)_2(CHPh)$ in 100 mL THF werden bei -78 °C tropfenweise mit einer Lösung von 1.2 mmol Di-(*R*)-1'-phenylethylimidazolin-2-yliden versetzt. Es wird im Laufe von 5 h langsam auf Raumtemperatur erwärmt und anschließend das Lösungsmittel entfernt. Man extrahiert das Rohprodukt mit einem Gemisch aus 2mL Toluol und 25 mL Pentan und fällt das Produkt bei - 78 °C aus dieser Lösung aus.
Ausbeute: 0.74 mmol (74 % d. Th.)
**EA** für $C_{44}H_{59}Cl_2N_2PRu$: calcd C 64.53, H 7.27, N 3.42; found C 64.58, H 7.34, N 3.44.
**$^1$H NMR** ($CD_2Cl_2$/ 25 °C): δ = 20.19 (1H, d, $^3J_{PH}$ = 4.5 Hz, Ru=CH), 7.74 - 7.00 (15H, all m, CH of $C_6H_5$), 6.83 (1H, m, NC*H*MePh), 6.73 (1H, s, NCH), 6.70 (1H, s, NCH), 2.52 (1H, m, NC*H*MePh), 2.44 (3H, m, CH of $PCy_3$), 2.11 (3H, d, $^3J_{HH}$ = 6.8 Hz, NCH*Me*Ph), 1.82-1.12 (30H, all m, $CH_2$ of $PCy_3$)1.35 (3H, d, $^3J_{HH}$ = 6.8 Hz, NCH*Me*Ph).

**$^{13}$C NMR** ($CD_2Cl_2$ / 25 °C): δ = 292.7 (Ru=CH), 183.4 (d, $J_{PC}$ = 78 Hz, NCN), 151.8 (*ipso*-C of $C_6H_5$), 140.1 and 139.5 (*ipso*-C of NCHMe*Ph*), 129.5, 128.5, 128.3, 127.9, 127.5,127.4,127.2,126.6, and 126.1 (*o*-C, *m*-C, and *p*-C of $C_6H_5$) 119.8 and 118.4 (NCH), 57.4 and 56.2 (N*C*HMePh), 31.3(d, $J_{PC}$ = 17 Hz, ipso-C of $PCy_3$), 29.0 (s, *m*-C of $PCy_3$), 28.9 (s, *m*-C of $PCy_3$), 27.2 (d, $J_{PC}$ = 3.7 Hz, *o*-C of $PCy_3$), 27.0 (d, $J_{PC}$ = 3.7 Hz, *o*-C of $PCy_3$), 25.8 (s, *p*-C of $PCy_3$) 21.7 and 20.3 (NCH*Me*Ph).
**$^{31}$P NMR** ($CD_2Cl_2$ / 25 °C): δ= 38.1.

1i) Benzyliden-dichloro-(1,3-di-((*R*)-1'-naphthylethyl)imidazolin-2-yliden)-(tricyclohexylphosphin)-ruthenium

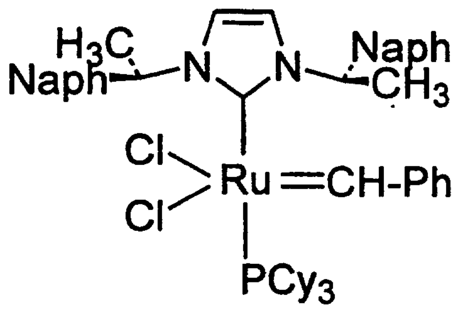

1 mmol $RuCl_2(PCy_3)_2(CHPh)$ in 100 mL THF werden bei -78 °C tropfenweise mit einer Lösung von 1.2 mmol Di-(*R*)-1'-naphthylethylimidazolin-2-yliden versetzt. Es wird im Laufe von 5 h langsam auf Raumtemperatur erwärmt und anschließend das Lösungsmittel entfernt. Man extrahiert das Rohprodukt mit einem Gemisch aus 2mL Toluol und 25 mL Pentan und fällt das Produkt bei - 78 °C aus dieser Lösung aus.
Ausbeute : 0.72 mmol (72 % d. Th.)
**EA** für $C_{52}H_{83}Cl_2N_2PRu$: calcd C 67.95, H 6.91, N 3.05; found C 68.09, H 7.02, N 3.04.
**$^{1}$H NMR** ($CD_2Cl_2$/ 25 °C): δ = 20.33 (1H, d, $^3J_{HH}$ = 5.4 Hz, Ru=CH), 8.88 (2H, d, $^3J_{HH}$ = 8.0 Hz, *o*-H of $C_6H_5$) 7.94 - 6.96 (17H, all m, CH of $C_6H_5$), , 6.70 (1 H, s, NCH), 6.61 (1H, s, NCH), 5.83 (1H, m, NC*H*MeNaph), 2.59 (1H, m, NC*H*MeNaph), 2.49 (3H, m, CH of $PCy_3$), 2.44 (3H, d, $^3J_{HH}$ = 6.8 Hz, NCH*Me*Naph), 1.95-1.01 (30H, all m, $CH_2$ of $PCy_3$)1.54 (3H, d, $^3J_{HH}$ = 6.8 Hz, NCH*Me*Naph).
**$^{13}$C NMR** ($CD_2Cl_2$/ 25 °C): δ = 298.4 (Ru=CH), 184.0 (d, $J_{PC}$ = 87 Hz, NCN), 152.3 (*ipso*-C of $C_6H_5$), 138.3 and 137.6 (*ipso*-C of NCHMe*Naph*), 134.3 -122.9 (*o*-C, *m*-C, and *p*-C of $C_6H_5$, CHMe*Naph*) 120.6 and 119.5 (NCH), 56.4 and 55.7 (N*C*HMeNaph), 32.5(d, $J_{PC}$ = 17 Hz, *ipso*-C of $PCy_3$), 30.1 (s, *m*-C of $PCy_3$), 30.0 (s, *m*-C of $PCy_3$), 28.1 (pseudo-t, $J_{PC}$ = 7.4 Hz, *o*-C of $PCy_3$), 26.8 (s, *p*-C of $PCy_3$) 24.0 and 22.7 (NCH*Me*Naph).
**$^{31}$P NMR** ($CD_2Cl_2$/25 °C): δ= 31.8.

2) Anwendung der erfindungsgemäßen Komplexverbindung bei der Olefin-Metathese

Die im folgenden aufgeführten Beispiele demonstrieren das Potential der erfindungsgemäßen Komplexverbindungen in der Olefin-Metathese. Der Vorteil dieser erfindungsgemäßen Komplexverbindungen verglichen mit phosphanhaltigen Komplexen liegt in der gezielten und kostengünstigen Variation der Reste R an den Stickstoffatomen der N-heterozyklischen Carbenliganden. Durch diese Maßschneiderung der erfindungsgemäßen Katalysatoren bezogen auf individuelle Eigenschaften der zu metathesierenden Olefine können Aktivität wie Selektivität der Reaktion gesteuert werden.

2a) Ringöffnende Metathese-Polymerisation (ROMP):

Als Beispiele dienen Norbomen, Cycloocten und funktionalisierte Norbomenderivate.

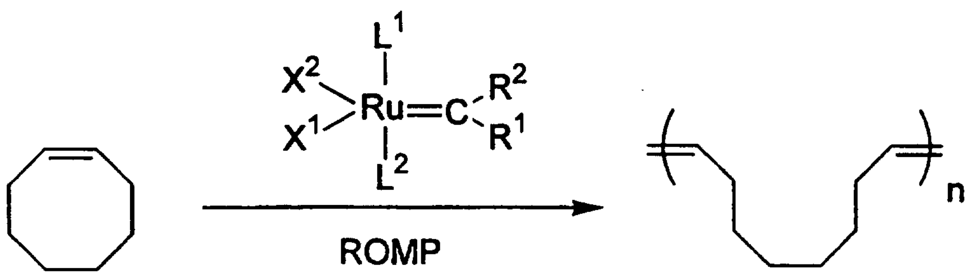


(4)

Typischer Reaktionsansatz für die Polymerisation von Cycloocten (bzw. Norbornen):

In eine Lösung von 3.6 mg (6.3 µmol) 1 in 0.5 ml Methylenchlorid wurden 410 µl (3.13 mmol) Cycloocten gegeben. Nach ca. 10 min hatte sich ein hochviskoses Gel gebildet, das nicht mehr gerührt werden konnte. Es wurde 1 ml Methylenchlorid zugesetzt. Diese Prozedur wurde immer dann wiederholt, wenn keine Rührerleistung mehr vorhanden war (insgesamt 3 ml Methylenchlorid). Nach 1 h wurden 5 ml Methylenchlorid zugegeben, dem geringe Mengen von tert-Butylether und 2,6-Di-tert-butyl-4-methylphenol zugesetzt wurden. Nach weiteren 10 min wurde die Lösung in einen hohen Überschuß von Methanol langsam eingetropft, filtriert und im Hochvakuum über mehrere Stunden getrocknet.
Ausbeute: 291 mg (2.64 mmol = 84.3 % d. Th.)

Tabelle 1.

| Polymerisation von Norbornen und Cycloocten | | | | | |
|---|---|---|---|---|---|
| Beispiel | Komplex | Monomer | Verhältnis [Monomer] / [Kat.] | Reaktionszeit **t** | Ausbeute |
| 2.1a | 1 | Norbornen | 100 : 1 | 1 min | 91 % |
| 2.1b | 5 | Norbornen | 100 : 1 | 1 min | 92 % |
| 2.1c | 1 | Cycloocten | 500 : 1 | 1 h | 84 % |
| 2.1d | 1 | Cycloocten | 500 : 1 | 2 h | 97 % |
| 2.1e | 5 | Cycloocten | 500 : 1 | 1 h | 87 % |

Typischer Reaktionsansatz für die Polymerisation von funktionalisierten Norbornenderivaten:

Die Formel **VIII** veranschaulicht das Grundgerüst der in Tabelle 2 eingesetzten Norbornenderivate.

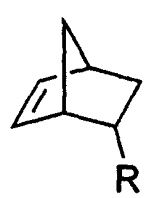

**VIII**

Zu einer Lösung von 3.6 mg (6.3 µmol) 1 in 0.2 ml Methylenchlorid wurden 0.3 ml einer Lösung von 432 mg (3.13 mmol) 5-Carbonsäure-2-norbomen (Formel **VIII** mit R = $CO_2H$) in Methylenchlorid gegeben. Nach ca. 10 min hatte sich ein hochviskoses Gel gebildet, das nicht mehr gerührt werden konnte. Es wurden weitere 0.5 ml Methylenchlorid zugesetzt. Diese Prozedur wurde immer dann wiederholt, wenn keine Rührerleistung mehr vorhanden war. Nach 1 h wurden 5 ml Methylenchlorid zugegeben, dem geringe Mengen von tert-Butylether und 2,6-Ditert-butyl-4-methylphenol zugesetzt waren. Nach weiteren 10 min wurde die Lösung in einen hohen Überschuß von Methanol langsam eingetropft, filtriert und im Hochvakuum über mehrere Stunden getrocknet.
Ausbeute: 423 mg (3.06 mmol = 98.1 % d. Th.)
Die Reaktionen bei 50 °C erfolgten in analoger Weise in Dichlorethan statt in Methylenchlorid.

Tabelle 2.

| Polymerisation funktionalisierter Norbornenderivate | | | | | |
|---|---|---|---|---|---|
| Beispiel | Komplex | Rest R in Formel **VIII** | T [°C] | Reaktionszeit t | Ausbeute |
| 2.1f | 1 | $O_2CCH_3$ | 25 | 30 min | 99 % |
| 2.1g | 1 | $CH_2OH$ | 25 | 2 h | 15 % |
| 2.1h | 1 | $CH_2OH$ | 50 | 2 h | 18 % |
| 2.1i | 1 | CHO | 25 | 2 h | 36 % |
| 2.1k | 1 | CHO | 50 | 2 h | 52 % |
| 2.1l | 1 | $COCH_3$ | 25 | 2 h | 42 % |
| 2.1m | 1 | $COCH_3$ | 50 | 2 h | 67 % |
| 2.1n | 1 | $CO_2H$ | 25 | 2 h | 98 % |

Die Polymerisation von Norbornen erfolgte dabei in Sekundenfrist. Bei der Cyclooctenpolymerisation wurden innerhalb einer Stunde nahezu quantitative
Umsätze erhalten (Tabelle 1). Unterschiede bezüglich der Aktivität sind durch Einsatz verschiedener Komplexe unter verdünnten Bedingungen nachweisbar und zeigen die Abhängigkeit der Aktivität vom Substitutionsmuster der eingesetzten Carbenliganden. Die hohe Stabilität und Toleranz gegenüber funktionellen Gruppen wird durch die Polymerisation funktionalisierter Norbornenderivate mit Ester, Alkohol, Aldehyd, Keton oder/und Carbonsäure demonstriert (Tabelle 2). Dabei konnten Monomere der allgemeinen Formel VIII mit R = $CH_2OH$, CHO und $CO_2H$ erstmals polymerisiert werden.

2.2) Ringschluß-Metathese (RCM) von 1,7-Octadien:

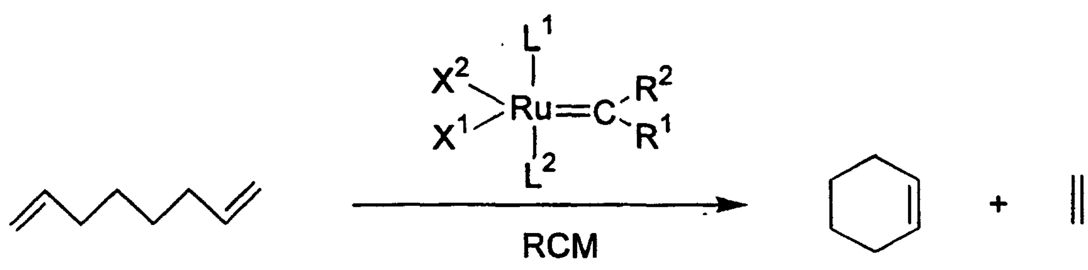


(5)

Typischer Reaktionsansatz für RCM von 1,7-Octadien:

Eine Lösung von 3.6 mg (6.3 µmol) 1 in 2 ml Dichlorethan wurde mit 46 µl (0.31 mmol) 9,7-Octadien versetzt, und der Reaktionsansatz wurde in ein 60 °C warmes Ölbad gegeben. Nach 1 h wurde das Reaktionsgemisch GC/MS-analytisch untersucht.

Tabelle 3.

| RCM von 1,7-Octadien (Octadien / Katalysator = 50:1) | | | | | |
|---|---|---|---|---|---|
| Beispiel | Komplex | Lösungsmittel | T [°C] | Reaktionszeit t | Ausbeute |
| 2.2a | 1 | Methylenchlorid | 25 | 5.5 h | 51% |
| 2.2b | 1 | Methylenchlorid | 25 | 24 h | 70 % |
| 2.2c | 1 | Dichlorethan | 60 | 1 h | 99 % |

Tabelle 3. (fortgesetzt)

| RCM von 1,7-Octadien (Octadien / Katalysator = 50:1) | | | | | |
|---|---|---|---|---|---|
| Beispiel | Komplex | Lösungsmittel | T [°C] | Reaktionszeit t | Ausbeute |
| 2.2d | <u>2</u> | Dichlorethan | 60 | 1 h | 99 % |
| 2.2e | <u>3</u> | Dichlorethan | 60 | 1 h | 99 % |
| 2.2f | <u>5</u> | Dichlorethan | 60 | 1 h | 99 % |

Das Potential in der Ringschluß-Metathese wurde durch die Reaktion von 1,7-Octadien zu Cyclohexen unter Freisetzung von Ethylen veranschaulicht (Tabelle 3). Mit <u>1</u> wurde nach 5.5 h eine Ausbeute von 51 % erzielt, bei 60 °C wurden mit allen eingesetzten erfindungsgemäßen Komplexverbindungen sogar quantitative Umsätze erzielt.

2.3) Metathese acyclischer Olefine

A) Metathese von 1-Octen:

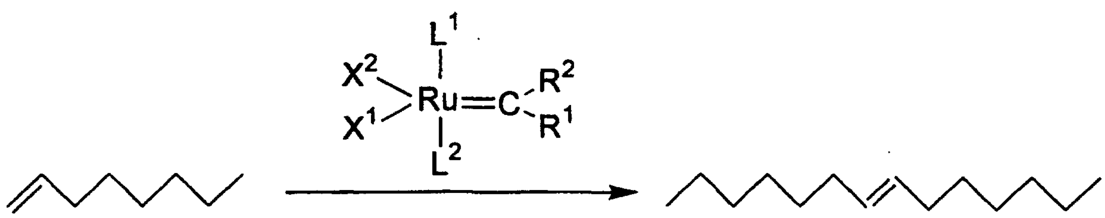

2

1-Octen

7-Tetradecen

(6)

Typischer Reaktionsansatz der Metathese von 1-Octen:

Eine Lösung von 3.6 mg (6.3 μmol) <u>1</u> in 2 ml Dichlorethan wurde mit 49 μl (0.31 mmol) 1-Octen versetzt, und der Reaktionsansatz wurde in ein 60 °C heißes Ölbad gegeben. Nach 3 h wurde das Reaktionsgemisch GC/MS-analytisch untersucht.

Tabelle 4.

| Homo-Metathese von 1-Octen (Octen / Katalysator = 50:1) | | | | | |
|---|---|---|---|---|---|
| Beispiel | Komplex | T [°C] | Reaktionszeit t | Umsatz von 1-Octen | Selektivität a |
| 2.3a | <u>2</u> | 60 | 1 h | 31% | 98% |
| 2.3b | <u>2</u> | 60 | 2 h | 58% | 97% |
| 2.3c | <u>1</u> | 60 | 1 h | 83% | 73% |
| 2.3d | <u>1</u> | 60 | 3 h | 97% | 63% |

a Die Selektivität gibt den Anteil an 7-Tetradecen gegenüber anderen metathetischen Produkten an

B) Metathese von Methyloleat:

$H_3C(CH_2)_7CH{=}CH(CH_2)_7COOMe$

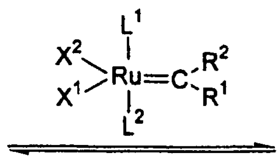

$H_3C(CH_2)_7CH{=}CH(CH_2)_7CH_3$

21 %

+

$MeOOC(CH_2)_7CH{=}CH(CH_2)_7COOMe$

21 %

**(7)**

<u>Typischer Reaktionsansatz für die Metathese von Methyloleat:</u>

Eine Lösung von 3.6 mg (6.3 µmol) **1** in 0.5 ml Dichlorethan wurde mit 1.06 ml (3.13 mmol) Methyloleat versetzt, und der Reaktionsansatz wurde für 15 h in ein 60 °C warmes Ölbad gegeben. Die GC/MS-Analyse ergab das in der Reaktionsgleichung (7) gezeigte Gleichgewicht von Metatheseprodukten.

Die Metathese von terminalen und innenständigen Olefinen wurde durch die Homo-Metathese von 1-Octen und Methyloleat nachgewiesen. Bei der Metathese von Methyloleat als nativem Rohstoff kann das thermodynamische Gleichgewicht innerhalb von 15 h mit Katalysator **1** bei einem Olefin : Katalysator-Verhältnis von 500 : 1 nahezu erreicht werden. Bei der Metathese von 1-Octen wurde 7-Tetradecen nicht in allen Fällen als einziges Reaktionsprodukt erhalten. Eine NMRspektroskopisch nachgewiesene Isomerisierung von 1-Octen zu 2-Octen und anschließende Olefin-Metathese ist für diesen Sachverhalt verantwortlich. Durch Homo- und Cross-Metathese von 1-Octen und 2-Octen wurde neben 7-Tetradecen auch als häufigstes Nebenprodukt 6-Tridecen und in geringen Mengen 6-Dodecen, 1-Hepten und 2-Nonen erhalten. Die Produktverteilung ist stark abhängig vom eingesetzten Katalysator. Im Fall von **2** wurde nahezu selektiv 7-Tetradecen erhalten; dagegen lieferte der aktivere Komplex **1** bei hohem Umsatz 7-Tetradecen nur mit einer Selektivität von 63 %. Als Nebenprodukt wurde im wesentlichen 6-Tridecen aus der Cross-Metathese von 1-Octen mit 2-Octen erhalten.

Ringöffnende Metathese Polymerisation (ROMP) von 1,5-Cyclooctadien

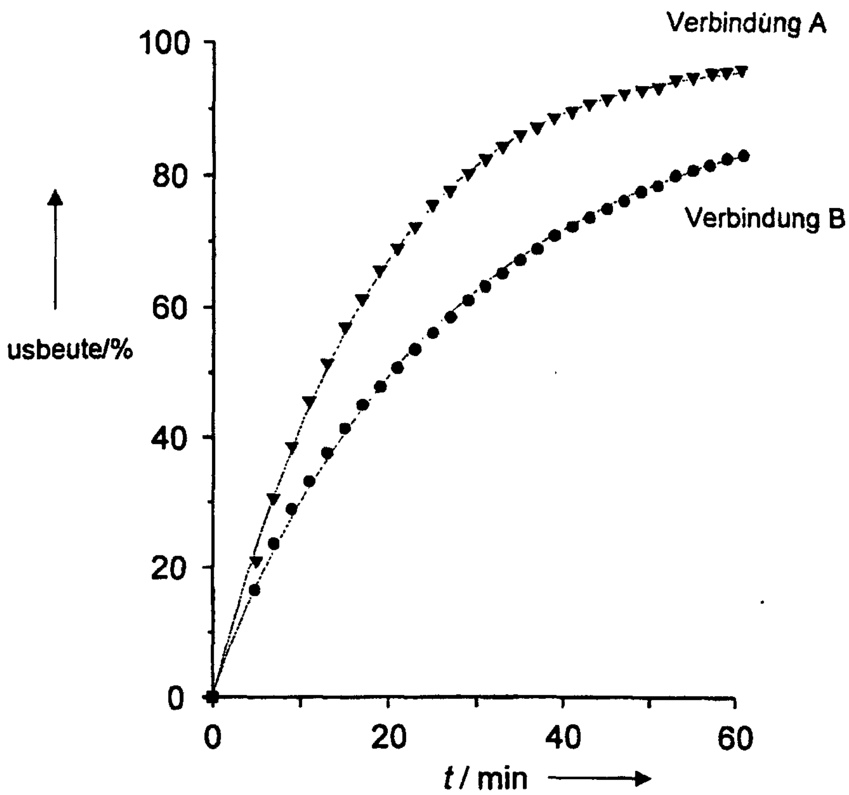


ROMP von 1,5-Cyclooctadien. NMR-Vergleich eines Ruthenium-Dicarben-Komplexes mit einem Ruthenium-Carben-Phosphan-Komplex. ($T$ = 25 °C; 1.70 µmol Katalysator in 0.55 mL $CD_2Cl_2$; [1,5-Cyclooctadien] / [Katalysator] = 250:1).

Verbindung A

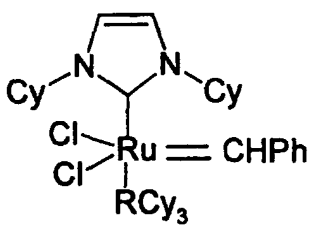

Verbindung B

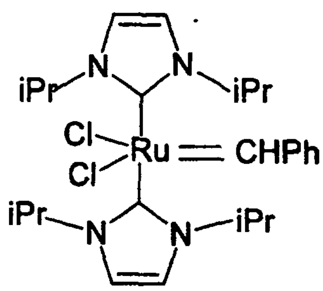

Gleiches gilt für ROMP von Cyclooctén:

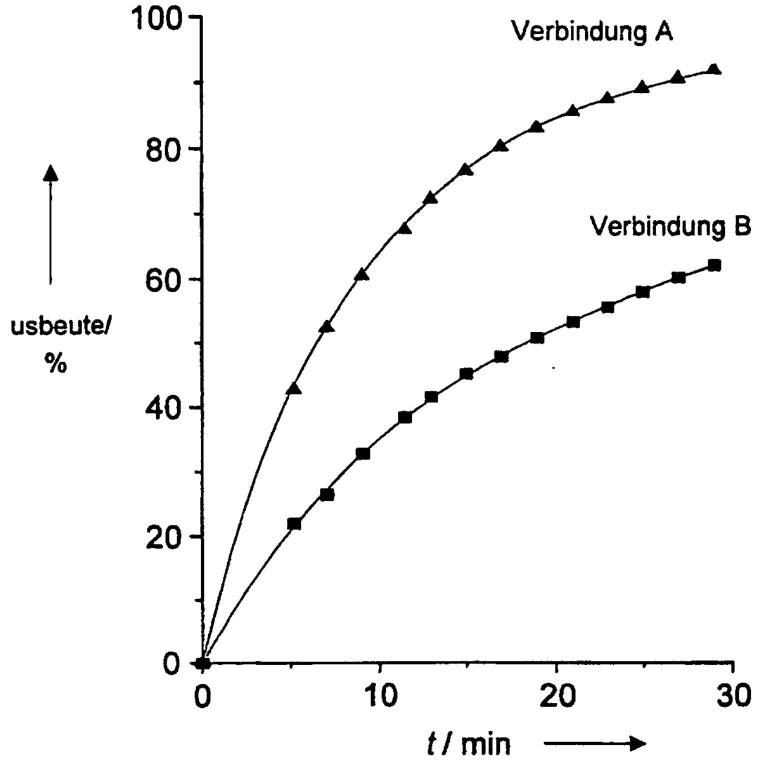


ROMP von Cycloocten. NMR-Kinetik eines Ruthenium-Dicarben-Komplexes mit einem Ruthenium-Carben-Phosphan-Komplex. ($T$ = 25 °C; 2.50 μmol Katalysator in 0.50 mL $CD_2Cl_2$; [Cycloocten] / [Katalysator] = 250:1).

Verbindung A

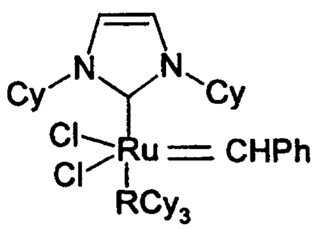

Verbindung B

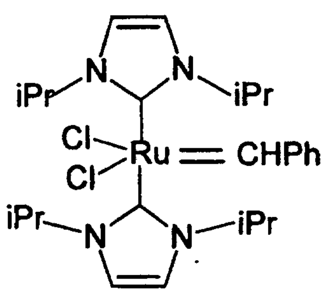

**Patentansprüche**

1. Komplexverbindung des Rutheniums der allgemeinen Strukturformel I,

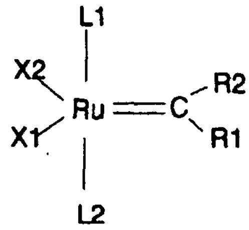

I

in der $X^1$ und $X^2$ gleich oder verschieden voneinander einen anionischen Liganden bedeuten,
in der $R^1$ und $R^2$ gleich oder unabhängig voneinander verschieden sind, aber auch einen Cyclus aufweisen können,
in der $R^1$ und $R^2$ für Wasserstoff oder/und für eine Kohlenwasserstoffgruppe stehen, wobei die Kohlenwasserstoffgruppen gleich oder unabhängig voneinander verschieden aus geradkettigen, verzweigten, cyclischen oder/und nicht cyclischen Resten aus der Gruppe von Alkylresten mit 1 bis 50 Kohlenstoffatomen, Alkenylresten mit 1 bis 50 Kohlenstoffatomen, Alkinylresten mit 1 bis 50 Kohlenstoffatomen, Arylresten mit 1 bis 30 Kohlenstoffatomen und Silylresten bestehen,
wobei in den Kohlenwasserstoff- oder/und Silylgruppen die Wasserstoffatome teilweise oder gänzlich durch eine Alkyl-, Aryl-, Alkenyl-, Alkinyl-, Metallocenyl-, Halogen-, Nitro-, Nitroso, Hydroxy-, Alkoxy-, Aryloxy-, Amino-, Amido-, Carboxyl-, Carbonyl-, Thio- oder/und Sulfongruppe einfach oder mehrfach, gleich oder unabhängig voneinander verschieden ersetzt sein können, in der der Ligand $L^1$ ein N-heterozyklisches Carben der allgemeinen Formeln II - V ist und in der der Ligand $L^2$ ein Phosphan ist,

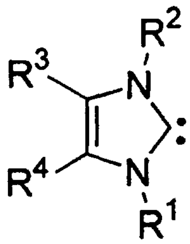

II

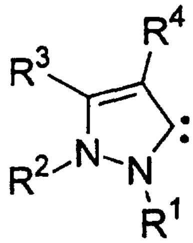

III

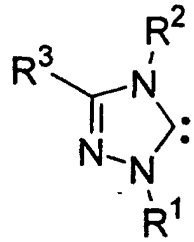

IV

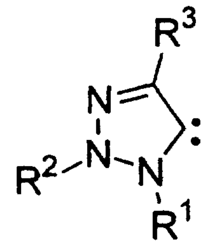

V

wobei $R^1$, $R^2$, $R^3$ und $R^4$ in den Formeln **II, III, IV** und **V** gleich oder verschieden für Wasserstoff oder/und für Kohlenwasserstoffgruppen stehen,
wobei die Kohlenwasserstoffgruppen aus gleichen oder verschiedenen, cyclischen, nicht cyclischen, geradkettigen oder/und verzweigten Resten aus der Gruppe von Alkylresten mit 1 bis 50 Kohlenstoffatomen, Alkenylresten mit 1 bis 50 Kohlenstoffatomen, Alkinylresten mit 1 bis 50 Kohlenstoffatomen und Arylresten mit 1 bis 30 Kohlenstoffatomen bestehen, bei denen gegebenenfalls mindestens ein Wasserstoff durch funktionelle Gruppen ersetzt sein kann, und wobei gegebenenfalls $R^3$ und $R^4$ für Halogen-, Nitro-, Nitroso-, Alkoxy-, Aryloxy-, Amido-, Carboxyl-, Carbonyl-, Thio- oder/und Sulfonylgruppen einfach oder mehrfach, gleich oder unabhängig voneinander verschieden stehen kann.

2. Komplexverbindung nach Anspruch 1 **dadurch gekennzeichnet, daß** die anionischen Liganden $X^1$ und $X^2$ gleich oder verschieden Halogenid, Pseudohalogenid, Tetraphenylborat, perhalogeniertes Tetraphenylborat, Tetrahalogenoborat, Hexahalogenophosphat, Hexahalogenoantimonat, Trihalogenomethansulfonat, Alkoxid, Carboxylat, Tetrahalogenoaluminat, Tertracarbonyl-Cobaltat, Hexahalogenoferrat(III), Tetrahalogenoferrat(III) oder/und Tetrahalogenopalladat(II) sind,
wobei Halogenid, Pseudohalogenid, Tetraphenylborat, perfluoriertes Tetraphenylborat, Tetrafluoroborat, Hexafluorophosphat, Hexafluoroantimonat, Trifluormethansulfonat, Alkoxid, Carboxylat, Tetrachloroaluminat, Tertracarbonyl-Cobaltat, Hexafluoroferrat(III), Tetrachloroferrat(III) oder/und Tetrachloropalladat(II) bevorzugt sind und wobei unter den Pseudohalogeniden Cyanid, Rhodanid, Cyanat, Isocyanat, Thiocyanat und Isothiocyanat bevorzugt sind.

**3.** Komplexverbindung nach Anspruch 1 und 2, **dadurch gekennzeichnet, daß** in den allgemeinen Formeln **II, III, IV** und **V** der Wasserstoff in den Kohlenwasserstoffgruppen $R^1$, $R^2$, $R^3$ und $R^4$ teilweise oder gänzlich durch Halogen-, Nitro-, Nitrose-, Hydroxy-, Alkoxy-, Aryloxy-, Amino-, Amido-, Carboxyl-, Carbonyl-, Thio-, Sulfonyl- oder/ und Metallocenylgruppen einfach oder mehrfach, gleich oder unabhängig voneinander verschieden ersetzt ist.

**4.** Komplexverbindung nach einem der Ansprüche 1 bis 3, **dadurch gekennzeichnet, daß** in den allgemeinen Formeln **II, III, IV** und **V** $R^3$ und $R^4$ ein anneliertes Ringsystem darstellt.

**5.** Komplexverbindung nach einem der Ansprüche 1 bis 4, **dadurch gekennzeichnet, daß** $L^1$ und $L^2$ einen Chelatliganden der allgemeinen Formel **VI**

$$L^1\text{-}Y\text{-}L^2$$

**VI**

ausbilden, wobei die mit **Y** bezeichneten Brückenglieder aus cyclischen, nicht cyclischen, geradkettigen oder/und verzweigten Resten aus der Gruppe von Alkylenresten mit 1 bis 50 Kohlenstoffatomen, Alkenylenresten mit 1 bis 50 Kohlenstoffatomen, Alkinylenresten mit 1 bis 50 Kohlenstoffatomen, Arylenresten mit 1 bis 30 Kohlenstoffatomen, Metallocenylen-, Borylen- und Silylenresten bestehen, bei denen gegebenenfalls mindestens ein Wasserstoff durch Alkyl-, Aryl-, Alkenyl-, Alkinyl-, Metallocenyl-, Halogen-, Nitro-, Nitrose-, Hydroxo-, Alkoxy-, Aryloxy-, Amino-, Amido-, Carboxyl-, Carbonyl-, Thio- oder/und Sulfonylgruppen, bevorzugt durch Alkyl-, Aryl- oder/und Metallocenylgruppen einfach oder mehrfach, gleich oder unabhängig voneinander verschieden substituiert ist.

**6.** Komplexverbindung nach einem der Ansprüche 1 bis 5, **dadurch gekennzeichnet, daß** die Liganden der allgemeinen Formeln **II, III, IV, V** oder/und **VI** zentrale, axiale oder/und planare Chiralität aufweisen.

**7.** Komplexverbindung nach einem der Ansprüche 1 bis 6, **dadurch gekennzeichnet, daß** in der allgemeinen Strukturformel I $R^1$ bis $R^2$ für Wasserstoff, substituierte oder/und nichtsubstituierte Alkyl-, Alkenyl- oder/und Arylreste stehen, daß $X^1$ und $X^2$ Halogenid-, Alkoxid- oder/und Carboxylationen sind oder/und daß $L^1$ für ein N-heterozyklisches Carben der allgemeinen Formel **II** steht.

**8.** Verfahren zur Herstellung von acyclischen Olefinen mit zwei oder mehr Kohlenstoffatomen oder/und von cyclischen Olefinen mit vier oder mehr Kohlenstoffatomen jeweils entsprechend der allgemeinen Formel **VII**

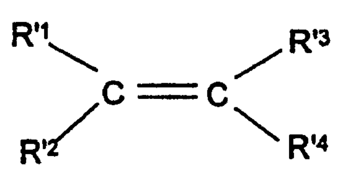

**VII**

aus acyclischen Olefinen mit zwei oder mehr Kohlenstoffatomen oder/und aus cyclischen Olefinen mit vier oder mehr Kohlenstoffatomen jeweils entsprechend der allgemeinen Formel **VII** durch Olefin-Metathese-Reaktion in Gegenwart mindestens eines Katalysators, **dadurch gekennzeichnet, daß**
ein Katalysator nach einem der Ansprüche 1 bis 7 eingesetzt wird und
daß $R'^1$, $R'^2$, $R'^3$ und $R'^4$ in der allgemeinen Formel **VII** für Wasserstoff oder/und Kohlenwasserstoffgruppen stehen, wobei die Kohlenwasserstoffgruppe aus gleich oder unabhängig voneinander verschieden geradkettigen, verzweigten, cyclischen oder/und nicht cyclischen Resten aus der Gruppe von Alkylresten mit 1 bis 50 Kohlenstoffatomen, Alkenylresten mit 1 bis 50 Kohlenstoffatomen, Alkinylresten mit 1 bis 50 Kohlenstoffatomen, Arylresten mit 1 bis 30 Kohlenstoffatomen, Metallocenyloder/und Silylresten besteht, bei denen gegebenenfalls mindestens ein Wasserstoff durch eine funktionelle Gruppe ersetzt sein kann,

wobei gegebenenfalls $R'^1$, $R'^2$, $R'^3$ und $R'^4$ für Halogen-, Nitro-, Nitrose-, Hxdroxy-, Alkoxy-, Aryloxy-, Amino-, Amido-, Carboxyl-, Carbonyl-, Thio-, Sulfonyl- oder/und Metallocenylgruppen einfach oder mehrfach, gleich oder unabhängig voneinander verschieden stehen.

**9.** Verfahren gemäß Anspruch 8, **dadurch gekennzeichnet, daß** in den eingesetzten Olefinen eine oder mehrere Doppelbindungen enthalten sind.

**10.** Verfahren gemäß Anspruch 8 oder 9, **dadurch gekennzeichnet, daß** $R'^1$, $R'^2$, $R'^3$ und $R'^4$ in den herzustellenden Olefinen der allgemeinen Formel **VII** paarweise, einfach oder mehrfach, gleich oder unabhängig voneinander verschieden einen Cyclus ausbilden.

**11.** Verfahren gemäß einem der Ansprüche 8 bis 10, **dadurch gekennzeichnet, daß** in den herzustellenden Olefinen der allgemeinen Formel **VII** der Wasserstoff in den Kohlenwasserstoffgruppen $R'^1$, $R'^2$, $R'^3$ und $R'^4$ teilweise oder gänzlich durch Halogen-, Silyl-, Nitro-, Nitrose-, Hydroxy-, Alkoxy-, Aryloxy-, Amino-, Amido-, Carboxyl-, Carbonyl-, Thio-, Sulfonyl- oder/und Metallocenylgruppen einfach oder mehrfach, gleich oder unabhängig voneinander verschieden ersetzt ist.

**12.** Verfahren nach einem der Ansprüche 8 bis 11, **dadurch gekennzeichnet, daß** der Prozeß mit oder ohne Lösungsmittel, jedoch vorzugsweise mit organischen Lösungsmitteln, durchgeführt wird.

**13.** Verfahren nach einem der Ansprüche 8 bis 12 **dadurch gekennzeichnet, daß** das Verfahren unter Zusatz einer Brönstedt-Säure, vorzugsweise von HCl, HBr, HI, $HBF_4$, $HPF_6$ oder/und Trifluoressigsäure, durchgeführt wird.

**14.** Verfahren nach einem der Ansprüche 8 bis 12 **dadurch gekennzeichnet, daß** das Verfahren unter Zusatz einer Lewis-Säure, vorzugsweise von $BF_3$, $AlCl_3$ oder/und $ZnI_2$, durchgeführt wird.

## Claims

**1.** Complex of ruthenium of the structural formula I,

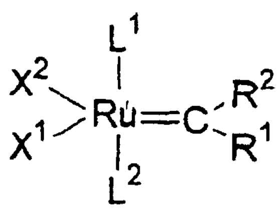

I

where $X^1$ and $X^2$ are identical or different and are each an anionic ligand,
$R^1$ and $R^2$ are identical or different and can also contain a ring, and $R^1$ and $R^2$ are each hydrogen or/and a hydrocarbon group, where the hydrocarbon groups are identical or different and are selected independently from among straight-chain, branched, cyclic or/and noncyclic radicals from the group consisting of alkyl radicals having from 1 to 50 carbon atoms, alkenyl radicals having from 1 to 50 carbon atoms, alkynyl radicals having from 1 to 50 carbon atoms, aryl radicals having from 1 to 30 carbon atoms and silyl radicals,
where one or more of the hydrogen atoms in the hydrocarbon or/and silyl groups can be replaced independently by identical or different alkyl, aryl, alkenyl, alkynyl, metallocenyl, halogen, nitro, nitroso, hydroxy, alkoxy, aryloxy, amino, amido, carboxyl, carbonyl, thio or/and sulphonyl groups,
the ligand $L^1$ is an N-heterocyclic carbene of the formulae II-V and the ligand $L^2$ is a phosphane,

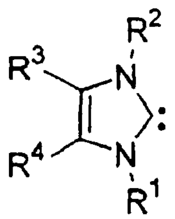

II

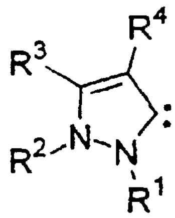

III

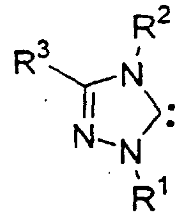

IV

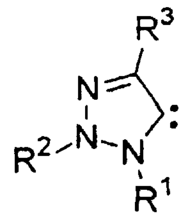

V

where $R^1$, $R^2$, $R^3$ and $R^4$ in the formulae II, III, IV and V are identical or different and are each hydrogen or/and a hydrocarbon group,
where the hydrocarbon groups comprise identical or different, cyclic, noncyclic, straight-chain or\and branched radicals selected from the group consisting of alkyl radicals having from 1 to 50 carbon atoms, alkenyl radicals having from 1 to 50 carbon atoms, alkynyl radicals having from 1 to 50 carbon atoms and aryl radicals having from 1 to 30 carbon atoms, in which at least one hydrogen may be replaced by functional groups, and where one or both of $R^3$ and $R^4$ may be identical or different halogen, nitro, nitroso, alkoxy, aryloxy, amido, carboxyl, carbonyl, thio or/and sulphonyl groups.

**2.** Complex according to Claim 1, **characterized in that** the identical or different anionic ligands $X^1$ and $X^2$ are each halide, pseudohalide, tetraphenylborate, perhalogenated tetraphenylborate, tetrahaloborate, hexahalophosphate, hexahaloantimonate, trihalomethanesulphonate, alkoxide, carboxylate, tetrahaloaluminate, tetracarbonylcobaltate, hexahaloferrate(III), tetrahaloferrate(III) or/and tetrahalopalladate(II),
with preference being given to halide, pseudohalide, tetraphenylborate, perfluorinated tetraphenylborate, tetrafluoroborate, hexafluorophosphate, hexafluoroantimonate, trifluoromethanesulphonate, alkoxide, carboxylate, tetrachloroaluminate, tetracarbonylcobaltate, hexafluoroferrate (III), tetrachloroferrate(III) or\and tetrachloropalladate(II) and preferred pseudohalides being cyanide, thiocyanate, cyanate, isocyanate and isothiocyanate.

**3.** Complex according to Claim 1 or 2, **characterized in that** some or all of the hydrogen atoms in the hydrocarbon groups $R^1$, $R^2$, $R^3$ and $R^4$ in the formulae II, III, IV and V are replaced independently by identical or different halogen, nitro, nitroso, hydroxy, alkoxy, aryloxy, amino, amido, carboxyl, carbonyl, thio, sulphonyl or/and metallocenyl groups.

**4.** Complex according to any one of Claims 1 to 3, **characterized in that** $R^3$ and $R^4$ in the formulae II, III, IV and V form a fused-on ring system.

**5.** Complex according to any one of Claims 1 to 4, **characterized in that** $L^1$ and $L^2$ form a chelating ligand of the formula VI

$$L^1\text{-}Y\text{-}L^2$$

**VI**

where the bridges Y can comprise cyclic, noncyclic, straight-chain or/and branched radicals selected from the group consisting of alkylene radicals having from 1 to 50 carbon atoms, alkenylene radicals having from 1 to 50 carbon atoms, alkynylene radicals having from 1 to 50 carbon atoms, arylene radicals having from 1 to 30 carbon atoms, metallocenylene, borylene and silylene radicals in which one or more hydrogens may be replaced independently by identical or different alkyl, aryl, alkenyl, alkynyl, metallocenyl, halo, nitro, nitroso, hydroxy, alkoxy, aryloxy, amino, amido, carboxyl, carbonyl, thio or/and sulphonyl groups, preferably alkyl, aryl or/and metallocenyl groups.

**6.** Complex according to any one of Claims 1 to 5, **characterized in that** the ligands of the formulae II, III, IV, V or/and VI have central, axial or/and planar chirality.

**7.** Complex according to any one of claims 1 to 6, **characterized in that** $R^1$ and $R^2$ in the structural formula I are hydrogen, substituted or/and unsubstituted alkyl, alkenyl or/and aryl radicals, $X^1$ and $X^2$ are halide, alkoxide or/and carboxylate ions or/and $L^1$ is an N-heterocyclic carbene of the formula II.

**8.** Process for preparing acyclic olefins having two or more carbon atoms or/and cyclic olefins having four or more carbon atoms, in each case of the formula VII

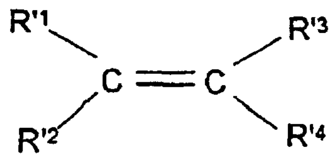

VII

from acyclic olefins having two or more carbon atoms or/and from cyclic olefins having four or more carbon atoms, in each case corresponding to the formula VII by an olefin metathesis reaction in the presence of at least one catalyst, **characterized in that** a catalyst according to any one of Claims 1 to 7 is used and $R'^1$, $R'^2$, $R'^3$ and $R'^4$ in the formula VII are hydrogen or/and hydrocarbon groups,
where the hydrocarbon groups are each selected independently from among straight-chain, branched, cyclic or/and noncyclic radicals of the group consisting of alkyl radicals having from 1 to 50 carbon atoms, alkenyl radicals having from 1 to 50 carbon atoms, alkynyl radicals having from 1 to 50 carbon atoms, aryl radicals having from 1 to 30 carbon atoms, metallocenyl or/and silyl radicals, in which one or more hydrogens may be replaced by a functional group,
where one or more of $R'^1$, $R'^2$, $R'^3$ and $R'^4$ may independently be identical or different halogen, nitro, nitroso, hydroxy, alkoxy, aryloxy, amino, amido, carboxyl, carbonyl, thio, sulphonyl or/and metallocenyl groups.

**9.** Process according to Claim 8, **characterized in that** one or more double bonds are present in the olefins used.

**10.** Process according to Claim 8 or 9, **characterized in that** $R'^1$, $R'^2$, $R'^3$ and $R'^4$ in the olefins of the formula VII to be prepared form, in pairs, one or more identical or different rings.

**11.** Process according to any one of Claims 8 to 10, **characterized in that** some or all of the hydrogen atoms in the hydrocarbon groups $R'^1$, $R'^2$, $R'^3$ and $R'^4$ of the olefins of the formula VII to be prepared are replaced independently by identical or different halogen, silyl, nitro, nitroso, hydroxy, alkoxy, aryloxy, amino, amido, carboxyl, carbonyl, thio, sulphonyl or/and metallocenyl groups.

**12.** Process according to any one of Claims 8 to 11, **characterized in that** the process is carried out in the presence or absence of solvents, but preferably in the presence of organic solvents.

**13.** Process according to any one of Claims 8 to 12, **characterized in that** the process is carried out with addition of a Brönsted acid, preferably HCl, HBr, HI, $HBF_4$, $HPF_6$ or/and trifluoroacetic acid.

**14.** Process according to any one of Claims 8 to 12, **characterized in that** the process is carried out with addition of a Lewis acid, preferably $BF_3$, $AlCl_3$ or/and $ZnI_2$.

## Revendications

**1.** Composé complexe du ruthénium de formule de structure générale I,

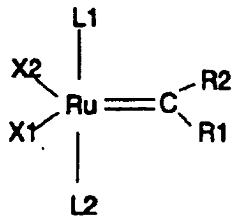

I

dans laquelle $X^1$ et $X^2$ sont identiques ou différents l'un de l'autre et signifient un ligand anionique,
dans laquelle $R^1$ et $R^2$ sont identiques ou indépendamment différents l'un de l'autre, mais peuvent également présenter un cycle,
dans laquelle $R^1$ et $R^2$ représentent hydrogène et/ou un groupe hydrocarboné, les groupes hydrocarbonés étant identiques ou indépendamment différents l'un de l'autre et constitués par des radicaux linéaires, ramifiés, cycliques et/ou non cycliques du groupe des radicaux alkyle comprenant 1 à 50 atomes de carbone, des radicaux alcényle comprenant 1 à 50 atomes de carbone, des radicaux alcynyle comprenant 1 à 50 atomes de carbone, des radicaux aryle comprenant 1 à 30 atomes de carbone et des radicaux silyle,
les atomes d'hydrogène dans les groupes hydrocarbonés et/ou silyle pouvant être remplacés partiellement ou totalement par un groupe alkyle, aryle, alcényle, alcynyle, métallocényle, halogène, nitro, nitroso, hydroxy, alcoxy, aryloxy, amino, amido, carboxyle, carbonyle, thio et/ou sulfone, de manière simple ou multiple, identique ou indépendamment différente l'un de l'autre, dans laquelle le ligand $L^1$ est un carbène N-hétérocyclique des formules générales II-V et
dans laquelle le ligand $L^2$ est un phosphane,

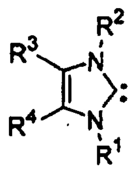 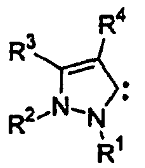 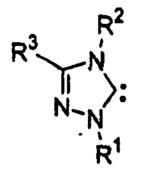 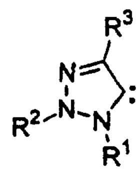

II III IV V

dans lesquelles $R^1$, $R^2$, $R^3$ et $R^4$ dans les formules II, III, IV et V sont identiques ou différents et représentent hydrogène et/ou des groupes hydrocarbonés,
les groupes hydrocarbonés étant constitués par des radicaux identiques ou différents, cycliques, non cycliques, linéaires et/ou ramifiés du groupe des radicaux alkyle comprenant 1 à 50 atomes de carbone, des radicaux alcényle comprenant 1 à 50 atomes de carbone, des radicaux alcynyle comprenant 1 à 50 atomes de carbone et des radicaux aryle comprenant 1 à 30 atomes de carbone, dans lesquels le cas échéant au moins un hydrogène peut être remplacé par des groupes fonctionnels et dans lesquelles $R^3$ et $R^4$ peuvent le cas échéant représenter des groupes halogène, nitro, nitroso, alcoxy, aryloxy, amido, carboxyle, carbonyle, thio et/ou sulfonyle, de manière simple ou multiple, identique ou indépendamment différente l'un de l'autre.

**2.** Composé complexe selon la revendication 1,
**caractérisé en ce que** les ligands anioniques $X^1$ et $X^2$ sont identiques ou différents et représentent halogénure, pseudohalogénure, tétraphénylborate, tétraphénylborate perhalogéné, tétrahalogénoborate, hexahalogénophosphate, hexahalogénoantimonate, trihalogénométhanesulfonate, alcoxyde, carboxylate, tétrahalogénoaluminate, cobaltate de tétracarbonyle, hexahalogénoferrate (III), tétrahalogénoferrate (III) et/ou tétrahalogénopalladate (II), où halogénure, pseudohalogénure, tétraphénylborate, tétraphénylborate perfluoré, tétrafluoroborate, hexafluorophosphate, hexafluoroantimonate, trifluorométhanesulfonate, alcoxyde, carboxylate, tétrachloroaluminate, cobaltate de tétracarbonyle, hexafluoroferrate (III), tétrachloroferrate (III) et/ou tétrachloropalladate (II) sont préférés et où on préfère, parmi les pseudohalogénures, le cyanure, le thiocyanure, le cyanate, l'isocyanate, le thiocyanate

et l'isothiocyanate.

3. Composé complexe selon la revendication 1 et 2, **caractérisé en ce que** dans les formules générales II, III, IV et V, l'hydrogène dans les groupes hydrocarbonés $R^1$, $R^2$, $R^3$ et $R^4$ est remplacé partiellement ou totalement par des groupes halogène, nitro, nitroso, hydroxy, alcoxy, aryloxy, amino, amido, carboxyle, carbonyle, thio, sulfonyle et/ou métallocényle, de manière simple ou multiple, identique ou indépendamment différente l'un de l'autre.

4. Composé complexe selon l'une quelconque des revendications 1 à 3, **caractérisé en ce que** dans les formules générales II, III, IV et V, $R^3$ et $R^4$ représentent un système cyclique annelé.

5. Composé complexe selon l'une quelconque des revendications 1 à 4, **caractérisé en ce que** $L^1$ et $L^2$ forment un ligand de chélate de formule générale VI

$$L^1\text{-Y-}L^2$$

**VI**

où les éléments de pont désignés par Y sont constitués par des radicaux cycliques, non cycliques, linéaires et/ou ramifiés du groupe des radicaux alkylène comprenant 1 à 50 atomes de carbone, des radicaux alcénylène comprenant 1 à 50 atomes de carbone, des radicaux alcynylène comprenant 1 à 50 atomes de carbone, des radicaux arylène comprenant 1 à 30 atomes de carbone, des radicaux métallocénylène, borylène et silylène,
dans lesquels le cas échéant au moins un hydrogène est substitué par des groupes alkyle, aryle, alcényle, alcynyle, métallocényle, halogène, nitro, nitroso, hydroxo, alcoxy, aryloxy, amino, amido, carboxyle, carbonyle, thio et/ou sulfonyle, de préférence par des groupes alkyle, aryle ou/et métallocényle, de manière simple ou multiple, identique ou indépendamment différente l'un de l'autre.

6. Composé complexe selon l'une quelconque des revendications 1 à 5, **caractérisé en ce que** les ligands des formules générales II, III, IV, V ou/ou VI présentent une chiralité centrale, axiale ou/et plane.

7. Composé complexe selon l'une quelconque des revendications 1 à 6, **caractérisé en ce que** dans la formule de structure générale I, $R^1$ et $R^2$ représentent hydrogène, des radicaux alkyle, alcényle et/ou aryle substitués ou non substitués, $X^1$ et $X^2$ sont des ions halogénure, alcoxyde ou/et carboxylate ou/et $L^1$ représentent un carbène N-hétérocyclique de formule générale II.

8. Procédé pour la préparation d'oléfines acycliques comprenant deux atomes de carbone ou plus ou/et d'oléfines cycliques comprenant quatre atomes de carbone ou plus, à chaque fois selon la formule générale VII

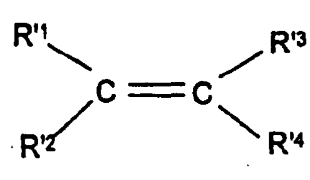

**VII**

à partir d'oléfines acycliques comprenant deux atomes de carbone ou plus ou/et à partir d'oléfines cycliques comprenant quatre atomes de carbone ou plus, correspondant à chaque fois à la formule générale VII par métathèse d'oléfines en présence d'au moins un catalyseur, **caractérisé en ce qu'**on utilise un catalyseur selon l'une quelconque des revendications 1 à 7 et **en ce que** $R'^1$, $R'^2$, $R'^3$ et $R'^4$ dans la formule générale VII représentent hydrogène ou/et des groupes hydrocarbonés,
le groupe hydrocarboné étant constitué par des radicaux linéaires, ramifiés, cycliques et/ou non cycliques identiques ou indépendamment différents les uns des autres du groupe des radicaux alkyle comprenant 1 à 50 atomes de carbone, des radicaux alcényle comprenant 1 à 50 atomes de carbone, des radicaux alcynyle comprenant 1 à 50 atomes de carbone, des radicaux aryle comprenant 1 à 30 atomes de carbone, des radicaux métallocényle et/ou silyle, dans lesquels le cas échéant au moins un hydrogène peut être remplacé par un groupe fonctionnel,

$R^{1'}$, $R^{'2}$, $R^{'3}$ et $R^{'4}$ représentant le cas échéant des groupes halogène, nitro, nitroso, hydroxy, alcoxy, aryloxy, amino, amido, carboxyle, carbonyle, thio, sulfonyle ou/et métallocényle, de manière simple ou multiple, identique ou indépendamment différente l'un de l'autre.

**9.** Procédé selon la revendication 8, **caractérisé en ce qu'**une ou plusieurs doubles liaisons sont contenues dans les oléfines utilisées.

**10.** Procédé selon la revendication 8 ou 9, **caractérisé en ce que** $R^{'1}$, $R^{'2}$, $R^{'3}$ et $R^{'4}$ dans les oléfines à préparer de formule générale VII forment par paire, de manière simple ou multiple, identique ou indépendamment différente l'un de l'autre, un cycle.

**11.** Procédé selon l'une quelconque des revendications 8 à 10, **caractérisé en ce que** dans les oléfines à préparer de formule générale VII l'hydrogène dans les groupes hydrocarbonés $R^{'1}$, $R^{'2}$, $R^{'3}$ et $R^{'4}$ est remplacé partiellement ou totalement par des groupes halogène, silyle, nitro, nitroso, hydroxy, alcoxy, aryloxy, amino, amido, carboxyle, carbonyle, thio, sulfonyle ou/ou métallocényle, de manière simple ou multiple, identique ou indépendamment différente l'un de l'autre.

**12.** Procédé selon l'une quelconque des revendications 8 à 11, **caractérisé en ce que** le procédé est réalisé avec ou sans solvant, de préférence toutefois avec des solvants organiques.

**13.** Procédé selon l'une quelconque des revendications 8 à 12, **caractérisé en ce que** le procédé est réalisé avec addition d'un acide de Brönstedt, de préférence HCl, HBr, HI, $HBF_4$, $HPF_6$ ou/et de l'acide trifluoroacétique.

**14.** Procédé selon l'une quelconque des revendications 8 à 12, **caractérisé en ce que** le procédé est réalisé avec addition d'un acide de Lewis, de préférence de $BF_3$, $AlCl_3$ ou/et $ZnI_2$.